# EUROPÄISCHE PATENTSCHRIFT

(11) **EP 2 491 959 B1**
(45) Veröffentlichungstag und Bekanntmachung des Hinweises auf die Patenterteilung: **17.08.2016**
(21) Anmeldenummer: 12153311.1
(22) Anmeldetag: 31.01.2012
(51) Int. Cl.: A61L 27/30, A61L 27/50, A61L 27/36

(54) **Beschichtetes biologisches Material mit verbesserten Eigenschaften für die Herstellung von Herzklappenprothesen**
Coated biological material having improved properties for the manufacture of heart valve prostheses
Matériau biologique revêtu doté de propriétés améliorées pour la fabrication de prothèses de valvule cardiaque

(30) Priorität: 24.02.2011 US 201161446054 P
(43) Veröffentlichungstag der Anmeldung: 29.08.2012
(73) Patentinhaber: Biotronik AG, 8180 Bülach (CH)
(72) Erfinder: Borck, Alexander, 91086 Aurachtal (DE); Rzany, Alexander, 90449 Nürnberg (DE)
(74) Vertreter: Randoll, Sören

(56) Entgegenhaltungen:
- WO-A2-2011/000357
- US-A1- 2006 193 885
- US-A1- 2008 086 205
- US-A1- 2009 169 871
- US-A1- 2010 106 233
- US-B1- 6 254 635

## Beschreibung

Der Ersatz von nicht mehr funktionsfähigen Herzklappen durch Herzklappenprothesen ist nach der koronaren Bypassoperation die am häufigsten durchgeführte Operation am menschlichen Herzen.

Dabei können grundsätzlich zwei verschiedene Typen von Herzklappenprothesen eingesetzt werden, mechanische oder biologische Herzklappenprothesen.

Mechanische Herzklappenprothesen und die damit verbundenen begleitenden Medikationen bringen erhebliche Einschränkungen für die Patienten mit sich. Patienten mit einer mechanischen Herzklappe müssen sich lebenslang einer Antikoagulationsbehandlung unterziehen und unterliegen damit dauerhaft einer erhöhten Gefährdung durch thromboembolische Komplikationen als auch durch Blutungen.

Um diese Nachteile und Schwierigkeiten zu umgehen, wurden biologische Herzklappenprothesen auf der Basis von humanem Gewebe (als Allo- oder Homograft) oder tierischem Gewebe (als Xenograft) entwickelt. Bei der Entwicklung von biologischen Herzklappenprothesen, insbesondere bei solchen Prothesen mit Klappensegeln aus biologischem Material tierischen Ursprungs (sog. Xenografts), kann auf eine dauerhafte Antikoagulation verzichtet werden. Allerdings neigen biologische Herzklappenprothesen zur Kalzifizierung, wobei die Verkalkung zum Funktionsverlust der Prothese führen kann. Es konnte gezeigt werden, dass trotz aller Sorgfalt und Vorsicht bei der Aufbereitung des biologischen Materials bereits geringe Rückstände von nicht vollständig denaturierten Proteinen oder Zellen nach der Implantation zu einer biologischen Antwort führen können. Im Ergebnis werden frühzeitige Kalzifizierungen des biologischen Klappenmaterials beobachtet. Die biologische Herzklappenprothese versagt und muss gegebenenfalls ausgetauscht werden. Neben der Kalzifizierung stellt auch die andauernde mechanische Belastung der Prothesen ein Problem dar, so dass biologische Herzklappenprothesen meist geringere Standzeiten aufweisen als mechanische Herzklappenprothesen. <hier Seite 2a einfügen>

Aufgabe der vorliegenden Erfindung ist es, einen oder mehrere Nachteile des Standes der Technik zu vermindern oder zu vermeiden. Insbesondere ist es eine Aufgabe der vorliegenden Erfindung biologisches Material für die Verwendung in biologischen Herzklappenprothesen bereitzustellen, welches lange Standzeiten der Herzklappenprothesen erlaubt. Die vorliegende Erfindung löst diese Aufgabe durch Bereitstellung von beschichtetem biologischem Material für die Herstellung von Herzklappenprothesen nach Anspruch 1, dadurch gekennzeichnet, dass die Oberfläche des biologischen Materials ganz oder teilweise mit einer Beschichtung bedeckt ist, die einen biokompatiblen anorganischen Werkstoff enthält oder daraus besteht.

Durch die Beschichtung mit einem biokompatiblen anorganischen Werkstoff ist das aus dem erfindungsgemäßen, beschichteten biologischen Material hergestellte Klappenmaterial sehr viel unempfindlicher gegen Kalzifizierung. Daneben sind auch Abstoßungsreaktionen nach Implantation von daraus gefertigten Herzklappenprothesen deutlich reduziert. Ggf. vorhandene immunogene Bestandteile des biologischen Materials sind durch die biokompatible anorganische Beschichtung vor einem direkten Kontakt mit dem Immunsystem des Empfängers oder Patienten geschützt. Darüber hinaus trägt die Beschichtung mit einem biokompatiblen anorganischen Werkstoff zur mechanischen Stabilisierung des biologischen Materials und von daraus hergestelltem Klappenmaterial gegen Abrieb bei, so dass insgesamt längere Standzeiten für biologische Herzklappenprothesen erzielt werden können, die erfindungsgemäßes, beschichtetes biologisches Material aufweisen oder daraus bestehen. In der Folge können jüngere Patientenpopulationen mit Herzklappenprothesen enthaltend das erfindungsgemäße beschichtete biologische Material versorgt werden, wobei eine Nachbehandlung erst deutlich später oder ggf. gar nicht mehr notwendig ist.

US 6254635 B1 offenbart ein beschichtetes biologisches Material. US 2008/0086205 A1 offenbart ein mit Polyphophazen beschichtetes biologisches Material. US 2010/0106233 A1 offenbart Gewebe, das mit Nanopartikeln beschichtet werden kann. WO 2011/000357 A2 offenbart ein Verfahren zur Beschichtung von biologischem Material.

Für die Zwecke der vorliegenden Erfindung wird unter dem Begriff "biologisches Material" solches biologisches Material verstanden, welches für die Herstellung von Herzklappenprothesen verwendet werden kann, insbesondere für die Herstellung von biologischen Herzklappenprothesen. Bevorzugt handelt es sich dabei um biologisches Material welches für die Herstellung von Klappensegeln von Herzklappenprothesen verwendet wird. Dabei ist das biologische Material biologischen Ursprungs, wird also aus Gewebe oder Bestandteilen von menschlichen oder tierischen Organismen oder Organen gewonnen oder ist durch menschliche oder tierische Zellen in vitro gebildet worden, beispielsweise mittels sog. "tissue engineering"-Techniken. Bevorzugt wird das biologische Material aus Perikard-, Herz-, Venen- und/oder Aortengeweben gewonnen, besonders bevorzugt wird das biologische Material aus Perikardgewebe, Herzklappengewebe, Venen- und/oder Aortenklappengewebe gewonnen. Da häufig nicht ausreichend biologisches Material menschlichen Ursprungs bereitgestellt werden kann, wird insbesondere biologisches Material xenogenen Ursprungs verwendet. Bevorzugt ist das biologische Material porcinen, bovinen oder equinen Ursprungs. So kann das biologische Material beispielsweise aus Herzklappen- oder Perikardmaterial aus dem Schwein gewonnen werden oder aus Perikardgewebe von Rindern oder Pferden. Es ist ebenfalls möglich das biologische Gewebe aus Venen- oder Aortengewebe zu gewinnen, wobei hier die Möglichkeit besteht ggf. humanes allogenes oder sogar autogenes Gewebe zu verwenden.

Um die gewünschte Konservierung und mechanische Stabilität zu gewährleisten und um die Gefahr von Kalzifizierungen sowie Abstoßungsreaktionen so gering wie möglich zu halten, kann das biologische Material behandelt worden sein. So wird häufig eine Fixierung, Konservierung und/oder Dezellularisierung durchgeführt. Entsprechende Techniken sind dem Fachmann bekannt. Bei der Vorbehandlung des biologischen Materials werden insbesondere Proteinbestandteile der extrazellulären Matrix vernetzt/fixiert, Zellen abgetötet/zerstört und immunogene, zelluläre Restbestandteile entfernt, so dass ein biologisches Material erhalten wird, welches im Wesentlichen aus fixierter, dezellularisierter extrazellulärer Matrix besteht. Bevorzugt umfasst oder besteht das biologische Material im Wesentlichen aus extrazellulärer Matrix, besonders bevorzugt aus dezellularisierter extrazellulärer Matrix. Diese extrazelluläre Matrix enthält bevorzugt überwiegend Kollagen- und Elastin-Bestandteile.

Das erfindungsgemäße beschichtete biologische Material zeichnet sich dadurch aus, dass die Oberfläche des biologischen Materials ganz oder teilweise mit einer Beschichtung enthaltend oder bestehend aus einem biokompatiblen anorganischen Werkstoff wie im Anspruch 1 bedeckt ist.

Eine Beschichtung im Sinne der Erfindung ist eine zumindest abschnittsweise Auftragung der Komponenten der Beschichtung auf die Oberfläche des biologischen Materials. Vorzugsweise wird die Oberfläche des biologischen Materials von der Beschichtung bedeckt, die nach Implantation der Herzklappenprothese unmittelbar in Kontakt kommt mit Bestandteilen des Immunsystems oder mit fließendem Blut des Empfängerorganismus. Eine Schichtdicke liegt vorzugsweise im Bereich von 10 nm bis 100 µm, besonders bevorzugt 50 nm bis 15 µm, ganz besonders bevorzugt von 100 nm bis 3 µm. Damit ist gewährleistet, dass die Beschichtung eine ausreichende Dicke aufweist, um die damit beschichtete Oberfläche nach erfolgter Implantation dauerhaft von einem direkten Kontakt mit Bestandteilen des Empfängerorganismus auszuschließen. Die Schichtdicke ist andererseits so gewählt, dass die mechanischen Eigenschaften des biologischen Materials beispielsweise in Bezug auf Flexibilität und Zähigkeit sowie ihre Eignung zur Verwendung als Klappenmaterial nicht wesentlich beeinträchtigt sind. Die Beschichtung kann direkt und unmittelbar auf die Oberfläche des biologischen Materials aufgetragen sein. Die Beschichtung kann beispielsweise mittels eines gepulsten Laserabscheideverfahrens, einem sogenannten "pulsed laser deposition "(PLD)-Verfahren erfolgen. Die Beschichtung kann als einschichtiges, aber auch als mehrschichtiges System erstellt werden, wobei unterschiedliche Schichten aus unterschiedlichen Werkstoffen bestehen können.

Die Beschichtung enthält oder besteht aus einem biokompatiblen, anorganischen Werkstoff. Ein biokompatibler, anorganischer Werkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes, der bei bestimmungsgemäßer Verwendung mit der Körperumgebung in Kontakt steht, ist dessen Biokompatibilität (Körperverträglichkeit). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Bevorzugt werden biokompatible anorganische Werkstoffe für die Beschichtung verwendet, die im Wesentlichen bioinert sind. Unter bioinerten Werkstoffen werden solche Werkstoffe verstanden, die nach der Implantation über die geplante Standzeit der Herzklappenprothese im Wesentlichen intakt bleiben und keine signifikante Biokorrosion zeigen. Als Prüfmedium zur Testung des Korrosionsverhaltens eines in Frage kommenden Werkstoffs dient künstliches Plasma, wie es nach EN ISO 10993-15:2000 für Biokorrosionsuntersuchungen vorgeschrieben ist (Zusammensetzung NaCl 6.8 g/l, CaCl₂ 0.2 g/l, KCl 0.4 g/l, MgSO₄ 0.1 g/l, NaHCO₃ 2.2 g/l, Na₂HPO₄ 0.126 g/l, NaH₂PO₄ 0.026 g/l). Eine Probe des zu untersuchenden Werkstoffs wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Tagen bis zu mehreren Monaten oder Jahren werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen. Ein Werkstoff gilt insbesondere dann als bioinert, wenn der Werkstoff in oben genanntem Test nach Ablauf von 12 Monaten zu weniger als 10% korrodiert ist.

Der biokompatible, anorganische Werkstoff kann beispielsweise aus einem Metall, einer Metalllegierung, einem Polymer, einem keramischen Werkstoff oder amorphem Kohlenstoff bestehen oder ein solches Material umfassen. Unter einer Legierung wird vorliegend ein metallisches Gefüge verstanden, dessen Hauptkomponente ein Metall, insbesondere Eisen, Tantal, Titan, Nickel, Cobalt, Chrom oder Molybdän ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Biokompatible Metalle und Metalllegierungen für die Beschichtung beinhalten beispielsweise Edelstähle (zum Beispiel 316L), Co/Cr-Legierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, Ti-Al6V4 oder TiAl6Nb7), Nickel oder Nickellegierungen, Tantal oder Tantallegierungen, Goldlegierungen und/oder amorphen Kohlenstoff, sogenanntes diamond-like carbon oder DLC Der biokompatible, anorganische Werkstoff ist ausgewählt aus Tantal, Nickel, Titan, Nitinol, Edelstahl, Legierungen daraus, Co/Cr-Legierungen und/oder amorphem Kohlenstoff mit einem sp² Hybridisierungsanteil von 30% bis 70%, beziehungsweise einem sp³ Hybridisierungsanteil von 70% bis 30% (sogenanntes diamond-like carbon oder DLC) der Wasserstoffgehalt dieser Schichten beträgt dabei zwischen 10% und 65%. Solche amorphen Kohlenstoffschichten (DLC) besitzen hervorragende mechanische und chemische Eigenschaften und weisen eine hohe Biokompatibilität auf. Aufgrund der Kombination von hoher Härte, niedrigem Reibwert und chemischer Beständigkeit eignet sich diese Schichtklasse ideal für den Einsatz in der Medizintechnik. Der Reibwert liegt beispielsweise um einen Faktor 3 bis 10 niedriger als übliche Bauteilmaterialien wie Stahllegierungen oder auch keramische Hartstoffschichten. Die Kombination aus hoher Härte und gleichzeitig niedrigsten Reibwerten ist ideal für biomedizinische Implantate wie z.B. Herzklappenprothesen. Dadurch lassen sich Abrieb (abrasiver Verschleiß) und Fressen (adhäsiver Verschleiß) verhindern oder mindestens merklich vermindern. Zudem werden DLC-Beschichtungen seit vielen Jahren im Bereich von Stents, mechanischen Herzklappen oder orthopädischen Implantaten erfolgreich klinisch anwendet.

Die vorliegende Erfindung bezieht sich auch auf Klappensegel für den Einsatz in einer Herzklappenprothese, wobei das Klappensegel aus erfindungsgemäßem beschichtetem biologischem Material besteht oder dieses enthält.

Ebenfalls Gegenstand der vorliegenden Erfindung ist eine Herzklappenprothese, die dadurch gekennzeichnet ist, dass diese erfindungsgemäßes beschichtetes biologisches Material enthält oder daraus besteht und/oder erfindungsgemäße Klappensegel umfasst.

Die vorliegende Beschreibung bezieht sich auch auf ein Verfahren zur Herstellung von erfindungsgemäßem beschichtetem biologischem Material. Das Verfahren ist insbesondere dadurch gekennzeichnet, dass das biologische Material mit einer Beschichtung enthaltend oder bestehend aus einem biokompatiblen anorganischen Werkstoff versehen wird. Dies wird dadurch erreicht, dass die Oberfläche des biologischen Materials mittels gepulster Laserabscheidung (sog. "pulsed laser deposition", PLD) teilweise oder ganz mit einer Beschichtung enthaltend oder bestehend aus einem biokompatiblen anorganischen Werkstoff bedeckt wird.

Die gepulste Laserabscheidung oder Laserablation (PLD) ist ein technisches Verfahren welches zur schonenden Aufbringung von metallischen Schichten auf Polymeren bereits bekannt ist. Die gepulste Laserabscheidung stellt einen Spezialfall eines physikalischen Gasphasenbeschichtungsverfahrens (PVD) dar. Bei der gepulsten Laserabscheidung wird mit hochenergetischen, kurzen Laserpulsen zu übertragender Werkstoff von einem Target durch Ablation in die Gasphase überführt und gerichtet in Form eines Plasmastrahls auf das zu beschichtende Substrat abgestrahlt und dort abgeschieden. Das Verfahren zeichnet sich durch die hohe Energiedichte des Laserpulses aus, was zu einer Anregung und Ablation des zu übertragenden Werkstoffs in einem sehr kleinen Volumen führt. Der Prozess der Ablation ist ein Nichtgleichgewichtsprozess, welcher im Gegensatz zum klassischen thermischen Verdampfen von Materialien nicht von den Dampfdrücken der einzelnen Komponenten abhängt. Die vom Laserpuls angeregten Atome des Werkstoffs gehen vielmehr unmittelbar in die Gasphase über, ohne einen Transfer von Energie zu Nachbaratomen. In der Konsequenz kommt es zu keinem Aufschmelzen des zu übertragenden Werkstoffs und zudem ist die Abscheiderate unabhängig von der chemischen Natur der einzelnen Werkstoffkomponenten. Zudem lässt sich das ablatierte Material aufgrund der geringen thermischen Energie auf biologischem Material als Substrat abscheiden. Eine Besonderheit, die die gepulste Laserabscheidung von vielen anderen Beschichtungstechniken unterscheidet, ist die Abscheidung im thermodynamischen Ungleichgewicht. Das bedeutet, dass anders als bei beispielsweise der thermischen Verdampfung, das zu übertragende Material nicht entsprechend seiner Verdampfungsenthalpie freigesetzt wird. Die Effektivität der Beschichtung ist unabhängig von der konkreten Substanz. Mit der gepulsten Laserabscheidung ist es deshalb auch möglich, Legierungen, wie sie im Target vorliegen, ohne Veränderung der Stöchiometrie auf das Substrat abzuscheiden. Durch den fokussierten Laserstrahl, die Abwesenheit thermischer Belastung und die gepulste Abscheidung können auch kleine Targets und selbst Beschichtungen als Quelle (Target) für den zu übertragenden Werkstoff eingesetzt werden, was insbesondere bei kostspieligen Proben zu einer Materialeinsparung führt.

Die Entwicklung der UV-Laser hat zu hohen Laserenergien von bis zu 1 J/Puls geführt, wobei einige Laser Pulsraten von bis zu 300 Hz erreichen. Durch die gepulste Laserabscheidung können bei Metallen Abscheideraten von mindestens 0.01 nm/Puls erzeugt werden. Entsprechend können Raten von 300 nm/Minute oder Schichtdicken von 1.5 µm in 5 Minuten erreicht werden, was insgesamt zu akzeptablen Beschichtungszeiten führt.

Bevorzugt wird im Verfahren für die gepulste Laserabscheidung ein gepulster Laser eingesetzt bei:
- einer Frequenz von 1 Hz bis 300 Hz, besonders bevorzugt bei einer Frequenz von 10 Hz bis 100 Hz;
   und/oder
- einer Pulsdauer von 0.1 ns bis 200 ns (ns = Nanosekunde), besonders bevorzugt von 1 ns bis 100 ns;
   und/oder
- einer Energiedichte von 0.01 J/cm² bis 100 J/cm², besonders bevorzugt von 0.1 J/cm² bis 30 J/cm², insbesondere von 1 J/cm² bis 15 J/cm²

Dabei können grundsätzlich Laser jeden Typs zum Einsatz kommen, insbesondere können für die gepulste Laserabscheidung gepulste Excimerlaser, CO₂-Laser oder Nd:YAG-Laser eingesetzt werden.

Im Verfahren erfolgt die gepulste Laserabscheidung im Vakuum. Unter einem Vakuum wird dabei ein Zustand verstanden, bei dem ein Gasdruck von weniger als 300 mbar herrscht. Bevorzugt erfolgt die gepulste Laserabscheidung bei einem Druck von nicht mehr als 1 mbar, besonders bevorzugt bei einem Druck von 0.1 mbar bis 10⁻¹⁰ mbar.

Bevorzugt wird im Verfahren die gepulste Laserabscheidung unter aseptischen Bedingungen durchgeführt. Dies stellt sicher, dass die Anforderungen an die Sterilität des hergestellten beschichteten biologischen Materials gewährleistet werden können.

In einer bevorzugten Ausführungsform des Verfahrens wird der biokompatible anorganische Werkstoff auf das biologische Material bis zu einer durchschnittlichen Schichtdicke von 1 nm bis 100 µm abgeschieden, besonders bevorzugt von 10 nm bis 15 µm, ganz besonders bevorzugt von 100 nm bis 3 µm.

Im Verfahren kann der zu übertragende biokompatible anorganische Werkstoff insbesondere aus Metall, einer Metalllegierung, einem Polymer, einem keramischen Werkstoff oder amorphem Kohlenstoff bestehen oder diesen umfassen, bevorzugt Tantal, Nickel, Titan, Nitinol, Edelstahl, Legierungen daraus, Co/Cr-Legierungen und/oder amorphem Kohlenstoff mit einem sp² Hybridisierungsanteil von 30% bis 70%, beziehungsweise einem sp³ Hybridisierungsanteil von 70% bis 30% (sogenanntes diamond-like carbon oder DLC).

Durch Wechsel der Beschichtungsquelle und damit des abzuscheidenden Werkstoffs, beispielsweise mittels einer Drehhalterung mit mehreren Beschichtungsquellen im Rezipienten, ist die Abscheidung eines Mehrschichtsystems auf dem biologischen Material möglich und somit eine weitere Möglichkeit gegeben für die Optimierung der physikalisch/chemischen Eigenschaften der Oberfläche des erfindungsgemäßen beschichteten biologischen Materials.

Bevorzugt wird das biologische Material mit oben beschriebenem Verfahren beschichtet, bevor es zur Herstellung von Klappensegeln oder bei der Montage von Herzklappenprothesen verwendet wird.

Das Verfahren erlaubt die direkte Beschichtung des biologischen Materials, welches die gewünschten mechanischen Eigenschaften von Herzklappen mitbringt, mit einer biokompatiblen anorganischen Schicht. Durch die anorganische Schicht wird zum einen die biologische Signatur des ggf. xenogenen biologischen Ausgangsmaterials zerstört und damit Abstoßungsreaktionen verhindert. Zum anderen wird durch die anorganische Oberfläche des beschichteten biologischen Materials dessen Kalzifizierung verhindert. Durch die schonende Beschichtung von biologischem Material mit biokompatiblen anorganischen Werkstoffen wie Metallen oder DLC-Schichten ist es möglich unter Beibehaltung der mechanischen Eigenschaften des biologischen Materials die Immunantwort des Körpers und das Problem der Kalzifizierung zu lösen. Die mechanischen Eigenschaften des biologischen Materials bleiben bei gleichzeitiger Optimierung der Oberfläche im Bezug auf Abrieb und biologisch/chemisch inertem Verhalten erhalten. Biologische Signaturen von Proteinen und Zellen werden von der anorganischen Beschichtung maskiert und der Immunantwort des Körpers des Empfängers entzogen. Nachdem der beschriebene Lösungsansatz mit keinen für die klinische Anwendung relevanten Einschränkungen hinsichtlich mechanischen Eigenschaften und Materialdicke verbunden ist, wird eine signifikante Verbesserung von biologischen Herzklappen auf der Basis von biologischem Material erreicht.

Figuren:

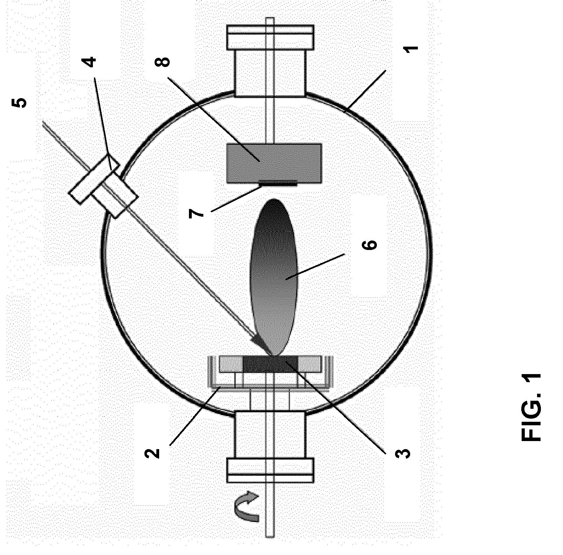

- FIG. 1: zeigt eine schematische Darstellung einer Beschichtungsanlage zur gepulsten Laserabscheidung (PLD) von biokompatiblem anorganischem Werkstoff auf biologischem Material.

Nachfolgend wird die Erfindung anhand von Ausführungsbeispielen näher erläutert.

In Figur 1 ist der grundsätzliche Aufbau einer Beschichtungsanlage zur gepulsten Laserabscheidung (PLD) von biokompatiblem anorganischem Werkstoff auf biologischem Material schematisch dargestellt. In einer Vakuumkammer 1 ist auf einem sich drehenden Karussell 2 das Target 3 befestigt. Das Target 3 enthält oder besteht aus dem zu übertragenden Werkstoff. Das Target 3 wird durch ein Fenster 4 in der Vakuumkammer 1 mit einem Laserstrahl 5 beschossen. Durch den Laserstrahl 5 wird zu übertragender Werkstoff vom Target 3 durch Ablation in die Gasphase überführt und gerichtet in Form eines Plasmastrahls 6 auf das zu beschichtende Substrat 7, also das zu beschichtende biologische Material abgestrahlt und abgeschieden, welches auf einer Befestigung 8 angebracht ist.

### Ausführungsbeispiel 1: Perikard mit Titanbeschichtung

Ein Ausführungsbeispiel ist die Beschichtung von aufbereitetem Perikardgewebe aus dem Schwein mit einer metallischen Schicht aus biokompatiblem Titan. Zur Abscheidung wird ein PLD-System bei Raumtemperatur mit einem 50 Hz Nd:YAG-Laser (532 nm Wellenlänge) bei einer Energiedichte von etwa 5 J/cm² verwendet. Als Beschichtungsquelle dient ein Blech aus reinem Titan (99.99% Reinheit). Der Restdruck in der Vakuumkammer beträgt 10⁻⁶ mbar. Der Abstand zwischen Beschichtungsquelle und zu beschichtendem Schweineperikard beträgt 3 cm. Die Abscheiderate beträgt unter diesen Bedingungen 0.025 nmBeschichtungspuls, d.h. 75 nm/Minute. Schichtdicken von mehr als einem Mikrometer lassen sich innerhalb von 15 Minuten erreichen.

Mit der gleichen Beschichtungsanlage und identischen Abscheideparametern lassen sich praktisch alle anderen Metalle mittels gepulster Laserabscheidung auf Perikardmaterial abscheiden.

### Ausführungsbeispiel 2: Perikard mit DLC-Beschichtung

Ein zweites Ausführungsbeispiel ist die Beschichtung mit biokompatiblem diamond-like Carbon (DLC), welches bereits bei künstlichen anorganischen Herzklappen erfolgreich klinische Anwendung findet. Für konventionelle DLC-Schichten ist ein zweistufiger Prozess notwendig. Im ersten Schritt wird durch ein Plasmaverfahren eine Kohlenwasserstoffschicht aufgebracht. Im weiteren Verlauf wird der Wasserstoffgehalt im Plasmapolymer verringert, bis der Kohlenstoff in der Diamantkonfiguration vorliegt. Die Prozesstemperaturen liegen bei weit über 100°C und sind für die Beschichtung von biologischem Perikardmaterial viel zu hoch. Durch gepulste Laserabscheidung sind Kohlenstoff- und Hartkohlenstoffbeschichtungen im Bereich der Raumtemperatur realisierbar.

Zur Abscheidung wird ein PLD-System bei Raumtemperatur mit einem 20 Hz Nd:YAG-Laser (1064 nm Wellenlänge) mit 25 ns Pulsdauer bei einer Energiedichte von etwa 10 J/cm² verwendet. Als Beschichtungsquelle dient ein Stab aus reinem Graphit (99.99% Reinheit), welcher während der Beschichtung mit 2 mm/min bewegt wird um Kraterbildung infolge von Materialabtrag zu minimieren. Der Restdruck in der Beschichtungskammer beträgt 10⁻⁶ mbar. Der Abstand zwischen Beschichtungsquelle und zu beschichtetem Schweineperikard beträgt 5 cm. Die Abscheiderate beträgt unter diesen Bedingungen 0.03 nm/Beschichtungspuls, d.h. 18 nm/Minute. Schichtdicken von mehr als 500 nm lassen sich innerhalb von 15 Minuten erreichen.

## Patentansprüche

1. Beschichtetes biologisches Material für die Herstellung von Herzklappenprothesen, **dadurch gekennzeichnet, dass** die Oberfläche des biologischen Materials ganz oder teilweise mit einer Beschichtung enthaltend oder bestehend aus einem biokompatiblen anorganischen Werkstoff bedeckt ist, wobei der biokompatible anorganische Werkstoff ausgewählt ist aus Tantal, Nickel, Titan, Nitinol, Legierungen daraus, Co/Cr-Legierungen und/oder amorphem Kohlenstoff mit einem sp² Hybridisierungsanteil von 30% bis 70%, beziehungsweise einem sp³ Hybridisierungsanteil von 70% bis 30%.

2. Beschichtetes biologisches Material nach Anspruch 1, wobei das biologische Material im Wesentlichen extrazelluläre Matrix umfasst oder daraus besteht, bevorzugt im Wesentlichen dezellularisierte extrazelluläre Matrix.

3. Beschichtetes biologisches Material nach Anspruch 1 oder 2, wobei das biologische Material aus Perikard-, Herz-, Venen- und/oder Aortengewebe gewonnen wird.

4. Beschichtetes biologisches Material nach einem der vorhergehenden Ansprüche, wobei das biologische Material humanen, porcinen, bovinen oder equinen Ursprungs ist, bevorzugt porcinen, bovinen oder equinen Ursprungs.

5. Beschichtetes biologisches Material nach einem der vorhergehenden Ansprüche, wobei die Beschichtung mit einem biokompatiblen anorganischen Werkstoff eine durchschnittliche Schichtdicke aufweist von 10 nm bis 100 µm, bevorzugt von 50 nm bis 15 µm, besonders bevorzugt von 100 nm bis 3 µm.

6. Beschichtetes biologisches Material nach einem der vorhergehenden Ansprüche, wobei der biokompatible anorganische Werkstoff amorphem Kohlenstoff mit einem sp² Hybridisierungsanteil von 30% bis 70%, beziehungsweise einem sp³ Hybridisierungsanteil von 70% bis 30% umfasst.

7. Klappensegel für die Verwendung in einer Herzklappenprothese, **dadurch gekennzeichnet, dass** das Klappensegel beschichtetes biologisches Material nach einem der Ansprüche 1 bis 6 umfasst oder daraus besteht.

8. Herzklappenprothese, **dadurch gekennzeichnet, dass** die Herzklappenprothese beschichtetes biologisches Material nach einem der Ansprüche 1 bis 6 und/oder ein oder mehrere Klappensegel gemäß Anspruch 7 umfasst.

## Claims

1. A coated biological material for the manufacture of heart valve prostheses, **characterised in that** the surface of the biological material is covered entirely or partially with a coating containing or consisting of a biocompatible inorganic material, wherein the biocompatible inorganic material is selected from tantalum, nickel, titanium, Nitinol, alloys thereof, Co/Cr alloys and/or amorphous carbon having an sp² hybridisation portion of 30% to 70%, or an sp³ hybridisation portion of 70% to 30%.

2. The coated biological material according to claim 1, wherein the biological material substantially comprises or consists of an extracellular matrix, preferably substantially decellularised extracellular matrix.

3. The coated biological material according to claim 1 or 2, wherein the biological material is obtained from pericardial, cardiac, venous and/or aortic tissue.

4. The coated biological material according to any one of the preceding claims, wherein the biological material is of human, porcine, bovine, or equine origin, preferably of porcine, bovine or equine origin.

5. The coated biological material according to any one of the preceding claims, wherein the coating with a biocompatible inorganic material has a mean layer thickness of 10 nm to 100 µm, preferably of 50 nm to 15 µm, particularly preferably of 100 nm to 3 µm.

6. The coated biological material according to any one of the preceding claims, wherein the biocompatible inorganic material comprises amorphous carbon having an sp² hybridisation portion of 30% to 70%, or an sp³ hybridisation portion of 70% to 30%.

7. A valvular leaflet cusp for use in a heart valve prosthesis, **characterised in that** the valvular leaflet comprises or consists of coated biological material according to any one of claims 1 to 6.

8. A heart valve prosthesis, **characterised in that** the heart valve prosthesis comprises coated biological material according to any one of claims 1 to 6 and/or one or more valvular leaflet according to claim 7.

## Revendications

1. Matériau biologique revêtu pour la fabrication de prothèses de valvules cardiaques, **caractérisé en ce que** la surface du matériau biologique est totalement ou partiellement recouverte avec un revêtement contenant, ou constitué, d'un matériau inorganique biocompatible, où le matériau inorganique biocompatible est choisi parmi du tantale, du nickel, du titane, du nitinol, ou des alliages de ceux-ci, d'alliages Co/Cr et/ou de carbone amorphe avec une proportion d'hybridation sp² de 30 % à 70 %, respectivement une proportion d'hybridation sp³ de 70 % à 30 %.

2. Matériau biologique revêtu selon la revendication 1, où le matériau biologique comprend principalement une matrice extracellulaire, ou en est constitué, de préférence, une matrice extracellulaire principalement décellularisée.

3. Matériau biologique revêtu selon la revendication 1, ou 2, où le matériau biologique est issu d'un tissu du péricarde, du coeur, de veines et/ou de l'aorte.

4. Matériau biologique revêtu selon l'une des revendications précédentes, où le matériau biologique est d'origine humaine, porcine, bovine ou équine, de préférence, d'origine porcine, bovine ou équine.

5. Matériau biologique revêtu selon l'une des revendications précédentes, où le revêtement avec un matériau inorganique biocompatible présente une épaisseur de couche moyenne de 10 nm à 100 µm, de préférence de 50 nm à 15 µm, de manière particulièrement préférée de 100 nm à 3 µm.

6. Matériau biologique revêtu selon l'une des revendications précédentes, où le matériau inorganique biocompatible comprend du carbone amorphe avec une proportion d'hybridation sp² de 30 % à 70 %, respectivement, une proportion d'hybridation sp³ de 70 % à 30 %.

7. Cuspide de valvule pour l'utilisation dans une prothèse de valvule cardiaque, **caractérisée en ce que** la cuspide de valvule comprend un matériau biologique revêtu selon l'une des revendications 1 à 6, ou en est constituée.

8. Prothèse de valvule cardiaque **caractérisée en ce que** la prothèse de valvule cardiaque comprend un matériau biologique revêtu selon l'une des revendications 1 à 6, et/ou une ou plusieurs cuspides de valvule selon la revendication 7.
